Europäisches Patentamt

European Patent Office

Office européen des brevets

(19)

(11) Veröffentlichungsnummer: **0 004 318**
**B1**

(12) **EUROPÄISCHE PATENTSCHRIFT**

(45) Veröffentlichungstag der Patentschrift: **16.02.83**

(51) Int. Cl.³: **A 61 B 10/00**

(21) Anmeldenummer: **79100699.2**

(22) Anmeldetag: **08.03.79**

(54) **Vorrichtung zur Zellgewinnung aus dem Endometrium.**

(30) Priorität: **23.03.78 DE 2812709**

(43) Veröffentlichungstag der Anmeldung:
**03.10.79 Patentblatt 79/20**

(45) Bekanntmachung des Hinweises auf die Patenterteilung:
**16.02.83 Patentblatt 83/7**

(84) Benannte Vertragsstaaten:
**BE CH FR GB IT LU NL SE**

(56) Entgegenhaltungen:
**FR - A - 2 345 131**
**US - A - 3 037 495**
**US - A - 3 168 092**
**US - A - 3 259 132**
**US - A - 3 500 819**
**US - A - 3 502 069**

(73) Patentinhaber: **Battelle-Institut e.V.**
**Am Römerhof 35 Postfach 900160**
**D-6000 Frankfurt/Main 90 (DE)**

(72) Erfinder: **Schlüter, Gert**
**Brunnenstrasse 41**
**D-6237 Liederbach (DE)**
Erfinder: **Stahl, Jürgen**
**Juchostrasse 3**
**D-6000 Frankfurt/Main (DE)**

(74) Vertreter: **Rupprecht, Klaus, Dipl.-Ing.**
**Am Römerhof 35**
**D-6000 Frankfurt (Main) 90 (DE)**

Courier Press, Leamington Spa, England.

## Vorrichtung zur Zellgewinnung aus dem Endometrium

Die Erfindung betrifft eine Vorrichtung zur Zellgewinnung aus dem Endometrium, bestehend im wesentlichen aus einem ausdehnbaren und in sich einstülpbaren Zellentnehmer in Form eines Ballons, der einerseits umlaufend mit der Öffnung eines Hohlzylinders und andererseits mit einem Zugelement verbunden ist und sich durch dessen Betätigung in den Hohlzylinder einziehen läßt wobei das Zugelement im ausgestülpten Zustand des Ballons mindestens teilweise aus dem Hohlzylinder herausragt, und einem Stutzen zur Einführung von Gas bzw. geeigneter Flüssigkeit.

Die Untersuchung des Endometriums spielt in der Diagnostik prämaligner und maligner Gewebeveränderungen eine entscheidende Rolle. Sie ist aber auch angezeigt für die Verlaufskontrolle therapeutischer Maßnahmen, für die Funktionsdiagnostik des Endometriums, welche die Beurteilung hormonell bedingter Einflüsse bzw. schwangerschaftsbedingter Veränderungen einschließt.

Die Untersuchung von Einzelzellen ist für die klinische Diagnostik insbesondere bei der Früherkennung von Krebskrankheiten sehr bedeutsam. Sie beruht auf dem Prinzip der Exfoliativzytologie, nämlich der Untersuchung spontan aus dem Krebsgewebe abgeschilferter oder durch einfach zur Abschilferung zu bringender Tumorzellen. Diese Zellen müssen gut beurteilbar und von normalen Zellen eindeutig unterscheidbar sein.

Die Probleme der Zelldiagnostik am Endometrium liegen z.B. darin, daß hormonell gesteuerte proliferierende Prozesse zelluläre Veränderungen im Endometrium provozieren können, die den Nachweis eventuell vorhandener atypischer Zellen erschweren. Hinzu kommt die schlechte Gewinnbarkeit endometrialer Zellen, die im wesentlichen nur durch eine verbesserte Probeentnahme vermieden werden kann.

Das Endometrium wird im allgemeinen durch Ausschabung gewonnen. Für die Beurteilung funktioneller Schleimhautbilder genügt die Gewinnung eines Gewebestreifens. Diese Methoden stellen einen operativen Eingriff dar. Es wurden aber auch zahlreiche andere Methoden für die Zellgewinnung entwickelt, die nicht nur unter Narkose durchgeführt werden müssen. Alle diese Entnahmeverfahren und -instrumentre erfordern jedoch eine geschickte Handhabung und sind mit Nachteilen belastet, die im wesentlichen mit ihrer Funktionsweise zusammenhängen.

Bei den bekannten Aspirations- und Spülmethoden (z.B. das Pistoletverfahren oder Gravlee Jet-wash) ist die Ergiebigkeit des ausgespülten bzw. aspirierten Materials abhängig von dem Abschilferungsgrad des Endometriums. Es können dabei nur Zellen gewonnen werden, die in lockeren bzw. losen Verbänden auf der Oberfläche des Endometriums liegen. Die Wirkung des Spüldrucks auf das Gewebe ist im Lumen des Uterus ungleichmäßig verteilt und nicht kontrollierbar. Ferner kann die Entnahme nicht aus allen Regionen erfolgen und die so gewonnenen Zellen besitzen im allgemeinen einen schlechten Erhaltungsgrad.

Auch das Abstreichen mit bürstenähnlichen Instrumenten (z.B. Medhosa-Kanüle und die in US—A—3 881 464 beschriebene Vorrichtung) bringt keine wesentlichen Vorteile, da diese Vorrichtungen in der Wirkung auf die Uterus-Schleimhaut ebenfalls nicht steuerbar sind und die Zellausbeute und die Ausschaltung einer Verletzung von der Geschicklichkeit des Anwenders abhängt. Ferner ist die Wiedergewinnung der Zellen schwierig, da Schleimhautfetzen in den Borsten der Vorrichtung hängenbleiben.

Andere Abstreichkanülen (z.B. Milan-Markey-Spirale) wirken auf das Endometrium sehr aggressiv, so daß Läsionen und Blutungen entstehen können. Auch diese Instrumente erlauben keine steuerbare Anwendung und garantieren keine repräsentative Zellentnahme.

Es wurden bereits Vorrichtungen zur Zellentnahme von Körperhöhlen vorgeschlagen, die am Einführungsende mit einem elastischen, durch ein Druckmittel ausdehnbaren Material versehen sind (FR—A—2 345 131 und US—A—3 259 132). Dadurch wird ein entsprechender Anpreßdruck auf die Wandung der Körperhöhle bewirkt und gleichzeitig eine schonende Zellgewinnung ermöglicht. Es ist auch eine Vorrichtung bekannt, die eine über die Öffnung eines zur Einführung dienenden Hohlzylinders gespannte elastische Membran aufweist (US—A—3 037 495). Alle diese Instrumente sind jedoch entweder konstruktiv aufwendig oder sie haben keine weitere Manipulationsmöglichkeit zur Unterstützung der Zellablösung. Auch Vorrichtungen, die im wesentlichen aus einem ausdehnbaren, mit der Öffnung des Hohlzylinders umlaufend verbundenen Ballon bestehen, weisen diese Nachteile auf (US—A—3 502 069, US—A—3 168 092 und US—A—3 500 819). Bei diesen Ausführungen läßt sich der Ballon unter Verwendung eines Druckmittels am Ort der Zellentnahme aus dem Hohlzylinder herausstülpen bzw. in den Hohlzylinder hineinziehen. Da der Ballon selbst zur Einführung dient, sind sie aber zur Zellentnahme aus engen Körperhöhlen nicht geeignet.

Die Aufgabe der vorliegenden Erfindung ist nun die Schaffung einer Vorrichtung, mit der bei einfacher Handhabung genügend gut erhaltenes und damit repräsentatives Zellmaterial gewonnen werden kann, ohne daß die Möglichkeit einer Verletzung des Gewebes besteht. Insbesondere sollte mit der Vorrichtung eine aus dem Endometrium bis in die Tubenwinkel reichende Zellgewinnung durchführbar sein.

Es hat sich nun gezeigt, daß sich diese Aufgabe mit einer Vorrichtung der eingangs genannten Art lösen läßt, wenn der Hohlzylinder als Einführungselement ausgebildet ist, das durch die Öffnung der Körperhöhle hindurch bis zur Stelle der Zellgewinnung hinschiebbar ist und wenn als Zugelement ein durch das Einführungselement hindurch geführter Zugstab verwendet ist, und wenn der Zugstab mit der inneren Fläche des Ballons durch ein, am oberen Teil des Zugstabs befestigtes, nicht starres Zwischenstück, z.B. ein Faden, verbunden ist. Die erfindungsgemäße Vorrichtung arbeitet nach dem Prinzip eines Abklatsch- bzw. Abreibeverfahrens.

Mit der erfindungsgemäßen Vorrichtung werden die Zellen an der Oberfläche des ballonartigen Zellentnehmers, z.B. aus Naturkautschuk oder aus einem thermoplastischen Elastomeren, gesammelt, Die Zellausbeute kann wesentlich erhöht werden, wenn der Ballon an seiner Oberfläche angerauht ist. Die Wirkungsweise ist dann ähnlich einer Abrasio (Ausschabung), so daß auch tiefere Zellschichten gewonnen werden können. Der Ballon wird mittels eines geeigneten Gases oder einer geeigneten Flüssigkeit von außen beschickt, so daß er aus dem Führungsrohr ausstülpen und sich den Innenwandungen des Organs lückenlos anlegen kann.

Der Zugstab dient insbesondere zum Einziehen des ausgestülpten Ballons in sich selbst und in das Einführungselement hinein. Dadurch wird verhindert, daß das gewonnene Zellmaterial beim Herausholen der Vorrichtung abgestreift und Fremdmaterialien eingeschleppt werden.

Ist der Zugstab hohl ausgebildet, so kann das Gas oder die Flüssigkeit an dem unteren Ende des Zugstabes eingeführt werden. In diesem Fall ist der Zwischenraum zwischen dem Hohlzylinder un dem Zugstab am oberen Teil des Hohlzylinders abgedichtet.

Weitere Merkmale, Vorteile und Anwendungsmöglichkeiten der Erfindung gehen aus der folgenden Schilderung weiterer Details sowie aus den beigefügten Abbildungen hervor.

Es zeigen in schematischer Vereinfachung:

Figur 1 ein Prinzipbild zur Erläuterung der erfindungsgemäßen Vorrichtung;

Figur 2 eine Ausführungsart, bei der das Gas bzw. die Flüssigkeit für die Ausdehnung des Ballons durch den Zwischenraum zwischen dem Einführungselement und dem Zugstab eingeleitet wird;

Figur 3 im Längsschnitt das Einführungselement mit hohl ausgebildetem Zustab (a) und die Ausgestaltung des dem Endometrium entgegengesetzten Endes des Hohlzylinders (b).

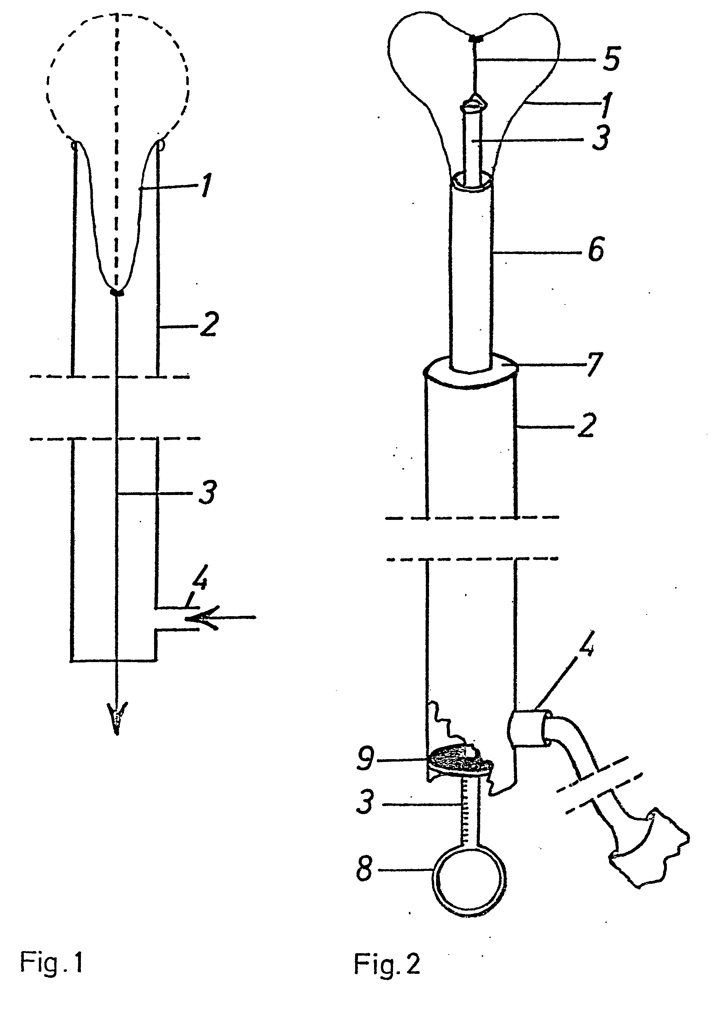

Aus Figur 1 geht hervor, daß der Ballon 1 mit der Öffnung des Hohlzylinders umlaufend kraftschlüssig und flüssigkeits- bzw. luftdicht verbunden ist. Die Verbindung kann gleichzeitig so ausgebildet sein, daß eine Entfernung des Ballons 1 nach der Verwendung möglich ist. Zur Einziehung des Ballons 1 in den Hohlzylinder 2 ist ein Zugelement 3 vorgesehen, das an der Innenseite des Ballons 1, möglichst an dessen oberem Pol, zentral befestigt ist. Bei Einführung von Gas bzw. einer geeigneten Flüssigkeit durch den Stutzen 4 kann der ballonartige Zellentnehmer 1 nach der Einführung in das Organ herausgestülpt werden und paßt sich an die Form des Untersuchungsortes an. Zur Verhinderung des Abweichens von Gas bzw. Flüssigkeit ist der Hohlzylinder 2 unten abgedichtet. Durch ein geeignetes Ventil an dem Stutzen 4 bzw. durch Aufsetzen einer Injektionsspritze kann das Volumen des eingeführten Gases oder der Flüssigkeit und somit der Umfang des Ballons verkleinert oder vergrößert werden, wodurch die Zellgewinnung von den Wandungen des Organs erleichtert wird.

Gemäß Figur 2 wird in dem Hohlzylinder 2 ein Zugstab 3 beweglich angebracht. Am oberen Teil ist der Zugstab 3 mit dem Zellentnehmer 1 mittels eines Fadens 5 verbunden. Die Verwendung eines kurzen Fadens verhindert, daß der Zugstab 3 im ausgedehnten Zustand des Zellentnehmers die Wandung des zu untersuchenden Organs verletzen kann.

Der Ballon 1 befindet sich vor der Einführung der Vorrichtung in dem etwas verjüngten Teil, d.h. im Einführungselement 6 des Hohlzylinders 2. Der Anschlag 7 kann die Einführungstiefe der Vorrichtung begrenzen; er kann beliebig einstellbar angebracht werden. Nach der Einführung der Vorrichtung in die Körperhöhle durch Schieben des Bedienungselementes 8 wird der Zugstab 3 und damit auch der Zellentnehmer 1 aus dem Einführungselement herausgeholt. Die Flüssigkeit bzw. das Gas wird ebenfalls über den Stutzen 4 durch den Zwischenraum zwischem dem Hohlzylinder 2 und dem Zugstab 3 eingeleitet. Eine im unteren Teil des Hohlzylinders 2 angebrachte Dichtung 9 verhindert, daß das Gas bzw. die Flüssigkeit entweicht. Der ausgedehnte Ballon 1 nimmt die Form des Organs an bzw. liegt an dessen Innenwandungen lückenlos an. Durch Drehen des Instrumentes, gleichzeitiges Verändern des Volumens des Ballons sowie durch Hin- und Herschieben des Zugstabes können zusätzliche reibende Bewegungen der Oberfläche des Zellentnehmers an der Schleimhaut erzeugt werden, wodurch die Zellausbeute wesentlich erhöht wird.

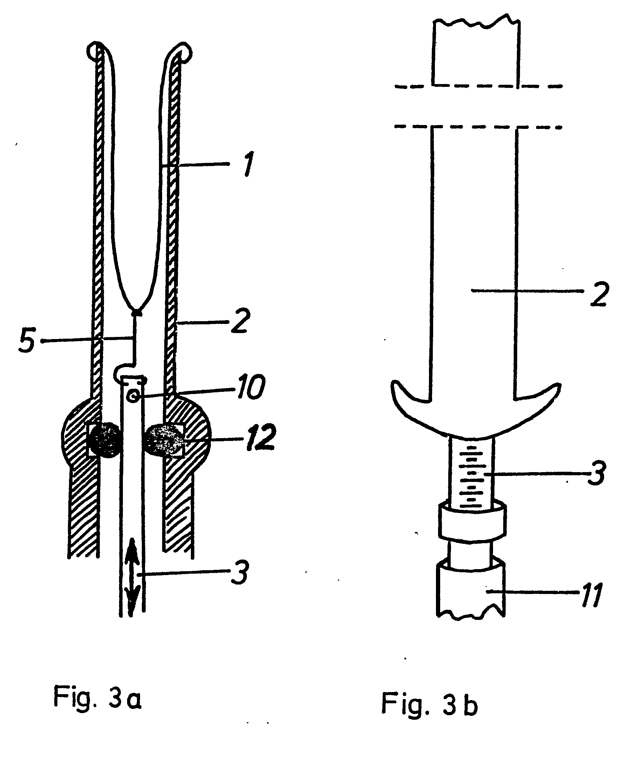

Der Zugstab kann auch hohl ausgebildet sein. Diese Ausführungsart ist in Figur 3 a) und b) dargestellt. In diesem Fall dient der Zugstab 3 gleichzeitig zur Einleitung des Mittels zur Dehnung des Ballons. An dem oberen Teil des Zugstabs 3 werden eine oder mehrere Austrittsöffnungen 10 vorgesehen (Figur 3 a)). Das untere Ende des Zugstabs 3 ist zweckmäßigerweise so ausgebildet, daß eine Injektionsspritze 11 angesetzt werden kann (Figur 3 b)). Zur Verhinderung des Entweichens von Gas bzw. Flüssigkeit ist ein Dichtungsring 12 vorgesehen, der in den Zwischenraum zwischen dem Hohlzylinder 2 und dem Zugstab 3 angebracht ist.

Der aus dem Hohlzylinder 2 herausragende Teil des Zugstabs 3 kann mit Markierungen versehen sein, die eine Ablesung der Einführungstiefe der Vorrichtung ermöglichen.

Die Wiedergewinnung des gewonnenen Zellmaterials aus den Wandungen des Ballons kann auf verschiedene Weise erfolgen. Nachdem der Ballon wieder entfaltet bzw. aufgeblasen ist, können die Zellen entweder über eine Kontakt- oder Abklatschvorrichtung direkt auf den Objektträger übertragen werden, wodurch eine Zuordnung des Zellmaterials zur Entnahmeregion ermöglicht wird. Man kann aber auch die gesamte Ballonoberfläche mittels einer Lösung, z.B. einer alkoholischen Fixierlösung, abspülen und das gesamte Zellmaterial durch Zentrifugieren gewinnen.

Mit Hilfe der erfindungsgemäßen Vorrichtung können außer Zellgewinnung auch therapeutische Maßnahmen, z.B. eine Blutstillung im Endometrium, eingeleitet werden. Durch Ausdehnung des Ballons mit einer Flüssigkeit, z.B. einer physiologischen Lösung, wird eine erhebliche Dehnung des Ballonkörpers erzielt, wobei offene Blutgefäße an den Wandungen des Organs komprimiert und die Blutung zum Stehen gebracht werden kann.

Es ist ebenfalls möglich, die Ausdehnung des Ballons durch ein Röntgenkontrastmittel zu erzielen, um den Sitz der Vorrichtung röntgenologisch überprüfen oder um wichtige Aufschlüsse über den inneren Aufbau des Organs erhalten zu können.

**Patentansprüche**

1. Vorrichtung zur Zellgewinnung aus dem Endometrium, bestehend im wesentlichen aus einem ausdehnbaren und in sich einstülpbaren Zellentnehmer in Form eines Ballons, der einerseits umlaufend mit der Öffnung eines Hohlzylinders, andererseits mit einem Zugelement verbunden ist und sich durch dessen Betätigung in den Hohlzylinder einziehen läßt wobei das Zugelement im ausgestülpten Zustand des Ballons (1) mindestens teilweise aus dem Hohlzylinder (6) herausragt, und einem Stutzen zur Einführung von Gas bzw. geeigneter Flüssigkeit, dadurch gekennzeichnet, daß der Hohlzylinder als Einführungselement (6) ausgebildet ist, das durch die Öffnung der Körperhöhle hindurch bis zur Stelle der Zellgewinnung hinschiebbar ist, und daß als Zugelement ein durch das Einführungselement (6) hindurch geführter Zugstab (3) verwendet ist, und daß der Zugstab (3) mit der inneren Fläche des Ballons (1) durch ein, am oberen Teil des Zugstabs befestigtes, nicht starres Zwischenstück, z.B. ein Faden (5) verbunden ist.

2. Vorrichtung nach Anspruch 1, dadurch gekennzeichnet, daß das dem Endometrium entgegengesetzte Ende des Zugstabes (3) mit Markierungen versehen ist, welche die Einführungstiefe definieren.

**Revendications**

1. Dispositif pour l'obtention de cellules à partir de l'endometrium, consistant essentiellement en un organe de prélèvement de cellules, extensible et enroulable sur lui-même, en forme de ballon, lequel est relié d'un côté, à circulation, avec l'ouverture d'un cylindre creux et, d'autre part, à un élément de traction, par actionnement duquel il peut être introduit dans le cylindre creux lorsque l'élément de traction, à l'état enroulé du ballon (1) fait saillie au moins en partie hors du cylindre creux (6), et comportant un raccord pour l'introduction de gaz ou d'un fluide approprié, dispositif caractérisé en ce que le cylindre creux est constitué comme élément d'introduction (6), qui peut être glissé, à travers l'ouverture de la cavité corporelle, jusqu'à l'emplacement de prélèvement de cellules, l'élément de traction étant constitué par une tige de traction (3), guidée à travers l'élément d'introduction (6) et qui est reliée avec l'intérieur du ballon (1) par une pièce intermédiaire non rigide, par exemple un fil fixé à l'extrémité de la tige de traction.

2. Dispositif suivant la revendication 1, caractérisé en ce que l'extrémité de la tige de traction (3) opposée à l'endomètre est pourvue de marques de repère qui définissent la profondeur d'introduction dans le corps.

**Claims**

1. Device for collecting cell material from the endometrium consisting essentially of an inflatable cell sampler which is retractable into itself and has the shape of a balloon which is connected on the one hand with the opening of a hollow cylinder and on the other hand with a retraction means for retracting the cell sampler into the hollow cylinder, the retraction means protruding at least partly from the hollow cylinder if the balloon is inflated and of a nozzle for introducing gas or a suitable liquid, characterized in that the hollow cylinder forms an introducing means (6) which can be pushed through the opening of the body cavity to the place of cell collection and that a drawbar (3) is used as the retraction means, said drawbar (3) being passed through the introducing means (6), and that the drawbar (3) is connected with the inner wall of the balloon (1) by a non-rigid intermediate means, e.g. a thread (5) which is attached to the upper part of the drawbar.

2. Device as claimed in claim 1, wherein the end of the drawbar (3) opposite to the endometrium is provided with marks defining the depth of introduction.

Fig.1          Fig.2

Fig. 3 a

Fig. 3 b